(19) **Europäisches Patentamt / European Patent Office / Office européen des brevets**

(11) **EP 3 420 891 B1**

(12) **EUROPEAN PATENT SPECIFICATION**

(45) Date of publication and mention
of the grant of the patent:
**20.03.2024 Bulletin 2024/12**

(21) Application number: **17755874.9**

(22) Date of filing: **23.02.2017**

(51) International Patent Classification (IPC):
**A61B 5/00** *(2006.01)*       **A61B 5/316** *(2021.01)*
**G06F 18/00** *(2023.01)*       **G06F 18/213** *(2023.01)*

(52) Cooperative Patent Classification (CPC):
**A61B 5/316; A61B 5/7253; G06F 18/00;**
**G06F 18/213;** A61B 5/352; G06F 2218/18

(86) International application number:
**PCT/ES2017/070104**

(87) International publication number:
**WO 2017/144763 (31.08.2017 Gazette 2017/35)**

(54) **SYSTEM FOR OBTAINING USEFUL DATA ASSOCIATED WITH HEART RATE VARIABILITY PATTERN**

SYSTEM ZUR GEWINNUNG VON NÜTZLICHEN DATEN IM ZUSAMMENHANG MIT EINEM HERZFREQUENZVARIABILITÄTSMUSTER

SYSTÈME POUR L'OBTENTION DE DONNÉES UTILES ASSOCIÉES AU MODÈLE DE VARIABILITÉ DE LA FRÉQUENCE CARDIAQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.02.2016 ES 201600164**

(43) Date of publication of application:
**02.01.2019 Bulletin 2019/01**

(73) Proprietor: **Universidad De Sevilla**
**41013 Sevilla (ES)**

(72) Inventor: **GAÑÁN CALVO, Alfonso Miguel**
**41092 Sevilla (ES)**

(74) Representative: **Jiménez Diaz, Rafael**
**Fernandez Palacio Abogados, S.L.P.**
**Plaza de la Magdalena nº 9-4º**
**41001 Sevilla (ES)**

(56) References cited:
WO-A1-2015/033244       WO-A1-2015/052609
WO-A1-2015/121679       WO-A1-2015/121679
US-A- 4 934 374       US-A1- 2005 171 447

- **ALFONSO M. GAÑÁN-CALVO ET AL: "Universal structures of normal and pathological heart rate variability", SCIENTIFIC REPORTS, vol. 6, no. 1, 25 February 2016 (2016-02-25), pages 1-11, XP055606420, DOI: 10.1038/srep21749**
- **ANTON BURYKIN et al.: "Dynamical density delay maps: simple, new method for visualising the behaviour of complex systems", BMC MEDICAL INFORMATICS AND DECISION MAKING, vol. 14, no. 1, 18 January 2014 (2014-01-18), page 6, XP021174448, LONDON, GB ISSN: 1472-6947**

**Description**

**TECHNICAL FIELD**

The invention that is proposed consists of a system for obtaining graphical data or the resulting multidimensional codes thereof, for characterizing and evaluating cyclic or quasi-periodical dissipative systems of any kind, whether natural or artificial, in a compact, universal, adaptable, and accessible manner. The invention can be preferably applied to the analysis of heart-rate variability data, and is essentially based on the following elements:

(i) Means for obtaining a general formulation of the normalized and sequential variability of time intervals between subsequent cycles, using a key of various parameters, which determine the particular algorithmic expression of said sequential variability. This expression can also be considered a general transform of the original numerical series of the time intervals. Furthermore, this transformation is univocal and establishes a sequential partition of the original series, selecting subsequent groups of N elements in an order previously established by the chosen key. Said groups can be considered vectors in an N-dimensional space.

(ii) Means for spatially representing, in N-dimensions, vectors or points generated by the transformation defined by the selected key. In particular, for N=4, the three dimensions and color can be used. In the case N=5, it is possible to use the three spatial dimensions, color, and a partition of the fifth dimension which can be represented dynamically, for example by means of a time sequence (video) of the three-dimensional representation corresponding to each interval of said partition, using time as the dimension for representing the fifth dimension.

(iii) Means for identifying common spatial patterns or correlations (clusters) between groups of points, such as crystallographic planes or axes, general surfaces, etc., obtained through the representations of a sufficient number of systems of the same nature.

(iv) Means for comparing an individual system of a certain nature with said common patterns, and determining of the presence of said common patterns in the individual system to be evaluated.

Contrary to any other systems using other transformations, such as the Fourier transform or the Wavelet transforms of any type, the proposed system is intrinsically adaptive and contemplates the accumulation of knowledge through experience. It furthermore allows using the instantaneous time scale or any other scale that may be locally selected, such as the main reference scale of the analysis; this allows identifying universal variability patterns, independent of other time scales or exogenous variables, which are inaccessible for other transformations such as the Fourier transform since they overlap all the time scales regardless of their sequence of appearance.

**BACKGROUND OF THE INVENTION**

The cyclic dissipative systems are characterized by specific time periods, whether they are constant or variable. Therefore, they belong to a special class of dynamic systems the degrees of freedom of which are critically restricted or limited. Unlike what occurs with generic dissipative dynamic systems, which show chaotic behavior, cyclic dissipative system attractors are well defined and are generally simple. Such systems are generally mechanical or biomechanical motors which exchange energy inputs or demands or work with the environment, whether it is an automobile engine, a wind turbine, or a heart. However, whereas constant time periods characterize both, artificial clocks and natural motors, designed specifically to minimize variability, or not being subjected to changes in demands, motors subject to variable demands also have variable time periods. This strategy is the most cost-effective way to assure the adaptive capacity of an organism (for example, an animal) or a machine (for example, a car) equipped with these motors.

The adaptive capacity with respect to changes in demands is probably the main priority of natural and artificial systems equipped with movement capabilities. These systems are inherently dissipative. On the other hand, all the artificial systems designed to work under constant demand also need to accelerate from a pause or decelerate until stopping. The degree of adaptability, or of how a system responds to a given demand, which in turn has its own characteristic time, designates its force, robustness, or health, and generally determines its survival.

Adaptable mobile systems generally experience periods during which they are in standby: for example, the idling speeds of internal combustion engines to prevent frequent starts and stops, the sleep of animals, etc. During these periods of inactivity, the system extends the characteristic time period exclusively to balance out internal dissipation. However, the complexity of the most adaptable mobile systems requires their internal cyclic motors to have a limited number of degrees of freedom. These limited degrees of freedom, which assure adaptability, are almost incompatible with constant time periods. The variability shown by a system in inactive mode openly reflects its internal characteristics and describes its compromises, which are often concealed under a general operation. For example, the two-stroke engines show strong variability when they are idling, and heart rate variability can be observed during deep sleep or in a situation of deep relaxation much better than during grilling exercise in healthy subjects.

An ample series of methods have been proposed for characterizing dissipative dynamic systems: description of strange attractors, Lyapunov exponents, entropy analysis, power laws, Fourier analysis, multidimensional phase portraits, etc. However, none of these methods provides in-depth information or univocal portraits for cyclic and adaptive dissipative systems due to the inherent nature thereof. There is no compact equivalent to a QR code or a graph to provide complete information about the nature, adaptability, state of health and internal characteristics of a cyclic dissipative system.

A very general case of systems with high variability and adaptability is that of living organisms equipped with a circulatory system and mobility. The circulatory system of the living organisms is an autonomous mechanical system delicately coupled with the respiratory system, and both have developed through evolution in response to the complex patterns of oxygen demand associated with movement. Circulatory health is based on adaptive capacity, which entails inherent variability. In the embodiment of the present invention described below, an N-dimensional graph calculated by means of the described method and representing heart rate variability shows two universal arrhythmic patterns as specific signs of health: one reflects the adaptive capacity of the heart, and the other reflects the harmony between heart-respiratory rhythm. Furthermore, at least three universal arrhythmic patterns are identified, the presence of which increases progressively in a manner that is proportionally detrimental to the two patterns of health, in certain pathological situations (myocardial infarction, heart failure, and recovery after sudden death). The presence of the identified universal arrhythmic structures, together with the position of the center of mass of the heart rate variability graph, provide an unprecedented quantitative assessment of the pathology-health gradient.

The heart is the first organic autonomous volumetric pump developed by nature, and it allowed the enormous leap that mobility entailed for living organisms. The basic functions of the heart are contractility and heart rate. Heart rate variability (HRV) represents a vital degree of freedom of the evolution of an autonomous organism with a circulatory system (Malik, 1996) that allows the immediate adaptive response to oxygen demand. These demands can have innumerable variability profiles. However, due to principles of economy, nature responds to demands with the creation of a limited number of structures or patterns, instead of giving different responses to the innumerable possible solutions. Therefore, one must ask, among other questions, to what extent do predetermined structures or patterns appear in HRV? Might some of those structures reveal general modes of adaptability (health) or of failure (pathology)? Can they appear combined? What would their general relative size and weight be with respect to the rest of the events? Are they complementary or antagonistic structures?

The respiratory system, which is complementary to the circulatory system in mobile organisms that live in the air, is also a volumetric pump (formed by the chest and lungs) with another characteristic time and an operation with a significant level of coupling to the cardiac system. The flexible relationship between heart rate and respiratory rate entails a specific HRV. The heart rate is also subjected to other endogenous influences, such as digestion, age and sex, biochemical mechanisms, or psychic activity, the characteristic times of which are decoupled from the autonomous control of the heart rate. When such influences reach or exceed external demands (including the circadian cycle), the organism may present pathological arrhythmias. However, those that are the most life-threatening are the ones that have rates greater than the respiratory rate. An example of the application of the present invention described herein shows that in the human species, there can be universal specific arrhythmic sequences (internal structure) as a co-evolutionary product of the autonomous (sympathetic-parasympathetic) nervous system, and that those sequences can be specific to a healthy heart or a diseased heart.

The need for precise and specific non-invasive tools for diagnosis and prognosis is a critical factor for advancement in medicine. Compact graphical representations of physiological systems have been of enormous help for doctors as regards the details and precision in diagnosis. Imaging internal systems and tissues (for example, ultrasound, computerized axial tomography, or magnetic resonance imaging (MRI)) has changed lives. As a result, in cardiac quantification (CQ), echocardiography (echoCG) has represented an extremely important development. Nevertheless, the series of electrocardiographic data provide irreplaceable complementary information that is inaccessible for echoCG in many aspects, such as the occurrence of patterns and temporary pathological behaviors, ectopic rhythms, etc. The reduction of the time series of a heart rate Holter recorder (HR) to a compact graphical representation (or graph) can have a clinical value that is complementary to that of the echoCG. Preliminary considerations are necessary: for the purpose of determining the occurrence of generic patterns reflecting normal or pathological characteristics and for improving their specificity, the graph must reduce to a minimum (i) the individual differences in weight/size, sex, age, etc., (ii) the long-term influences on heart rate, with times comparable to or greater than those of the respiratory rate, and (iii) both exogenous and endogenous influences, which are different from those of a cardiac or circulatory origin. The graphical representation that is sought must provide complete information provided by the sequence of all cardiac events (for example, the complete sequence beat by beat, or the RR intervals). After a thorough review, there is no evidence of there having been any representation with all those characteristics sought up until now.

Among the different approaches for studying HRV, including tools for the analysis of dynamic systems and chaos, multiscale entropy analysis, frequency domain analysis, and Markov chains, Poincaré maps of RR intervals provide compact graphs useful for the HRV analysis. The high stochastic heterogeneity HRV generally has is an indication of the existence of strong, presumably universal, internal structures. Under this premise, the most suitable tool for HRV would be a normalized time sequence analysis, as opposed to global stochastic tools or Fourier transforms. In fact, Fourier transform can definitively be excluded as a tool suitable for analyzing the universal variability patterns, since it essentially analyzes the content of fixed time (or frequency) scales in the entire recording, such that it mixes patterns of the same potential nature for high and low heart rates in the same HR.

The Poincaré representation (or recurrence maps) provides a direct sequence analysis of the RR series for identifying time patterns. However, the limitations of the representation of Poincaré recurrence maps in the two-dimensional (2D) spatial plane conceal a large part of the potential of graphs of this type.

Another type of representation is described, for example, in patent document US 4934374 (Ostlund et al.), where the represented object is the proximity between vectors defined by successive values of a signal with a given time delay as a function of time. The method described in this document is based on representing the time variable on both axes of a two-dimensional map, but the spatial representation of which continues to be planar (one-dimensional). Likewise, the representation described in said patent is based on a concept of "variability masked" by the modulus of a non-universal vector. Therefore, the influence of the instantaneous physical state of the patient will rule out any reliable form of detection of universal patterns.

Although this representation allows distinguishing ordered cyclic, quasi-periodical, or chaotic systems, it does not allow distinguishing complex sequences over time that are repeated or are specifically associated with certain pathologies. These characteristics are associated, for example, with specific arrhythmic sequences which appear, in any planar representation (as in the case of US 493374), overlapping and indistinguishable, the identification thereof in evaluation or diagnostic phases thereby being prevented.

Another example of a planar representation is the registration for the patent application published as US 2005/0171447 A1 (Esperer), which claims the use of the conventional two-dimensional Poincaré recurrence map. The author of that invention uses information in addition to that provided by the planar representation (the invention particularly uses the local density of points in each area of the plane that is represented) to correlate results with known pathologies. Nevertheless, this representation unavoidably conceals, by being superimposed on the plane, many complex multidimensional sequences which, again, the present invention does in fact show as a specific characteristic.

An example of multidimensional representation is shown in patent application PCT/GB2015/050429, where the represented object is a multidimensional vector consisting of successive values of any one signal coming from a quasi-periodical or cyclic system, separated by a fixed amount of time $\tau$ between every two successive values. The result is a more or less complex multidimensional orbit, the interpretation of which in terms of the identification of given complex multidimensional sequences may be impossible due to the inevitable possibility of there being multi-evaluated areas for one and the same sequence, which would prevent reconstructing said sequence. Even by varying the value of $\tau$ and producing a collection of different representations, the identification of complex sequences can be so difficult that a huge computational effort would be required to compact the information that is sought. Furthermore, said information must subsequently be correlated with the information about associated pathologies. In comparison, the selection of the values of the multidimensional vectors proposed in the present invention is the most suitable for offering the most compact and direct representation of complex sequences, which can be viewed directly without the need for any subsequent computational effort. Already known shape identification algorithms (which are not the object of the present invention) can then be applied to produce useful correlations between shape and pathology in a reliable, reproducible, compact, and quick manner.

Patent application WO2015033244A1 discloses a system for extracting a fetal heart rate from at least one maternal signal using a computer processor. The system includes sensors attached to a patient to receive abdominal ECG signals and a recorder and digitizer to record and digitize each at least one maternal signal in a maternal signal buffer. However, this system is unable to differentiate among patients with different heart function disturbances, and also lacks proper means for graphical representation of the arrhythmic structures derived from it.

Patent application WO2015052609A1 discloses an evaluating method for ECG signals which comprises the steps of: a) obtaining a multichannel ECG signal of a subject over a first predetermined time period by means of a multi-lead ECG device; b) extracting a plurality of first parameters from the multichannel ECG signal; c) evaluating the quality of the multichannel ECG signal based on the plurality of first parameters; and d) presenting an indicator for indicating the quality of the ECG signal via a user interface. However, the indicator obtained for this method cannot be represented uniquely for each patient, and it cannot produce a multidimensional map, such as a three-dimensional or four-dimensional graph, to represent complex potential arrhythmic structures which are specific for each patient and can be easily compared with universal patterns.

As a result of the above-described limitations of the prior art, in the present technical field it is necessary to develop new systems for the compact representation of the pattern of heart rate variability, which allow obtaining more detailed information than that which is generated by the methods used up until now. The present invention seeks to solve the aforementioned need by means of a novel system for the univocal and compact representation of cyclic or quasi-periodical dissipative systems of any nature which are of particular use in the analysis of cardiac patterns. Likewise, said system does not depend on the time of obtaining data, and by means of its steps of normalization and compaction, it allows finding associated patterns and the evolution thereof regardless of the time scale and in a multidimensional manner, which is not possible in other representations, such as those described in US 4934374, US 2005/0171447 or PCT/GB2015/050429. As further information on the invention published after the priority date of the present application, see: Gañán-Calvo et al., "Universal structures of normal and pathological heart rate variability", Sci. Rept. (20160225), vol. 6, no. 1, doi:10.1038/srep21749, pages 1-11.

## BRIEF DESCRIPTION OF THE INVENTION

A first object of the present invention relates to a system for obtaining data associated with a heart rate variability (HRV) pattern, comprising:

a1) means for measurement and data recording of an electrocardiogram, adapted for measuring and recording a number $M$ of consecutive time intervals $\{X_i\}_{i=1,\ldots,M}$ corresponding to cycles of one or more components of a heart "pQRSt" complex of an electrocardiogram, with a precision equal to or greater than 10% of the mean value of the cycle time, and $M$ being greater than 2;

a2) means for calculation and visual representation, adapted for performing calculations on the data recorded by the means for measuring and data recording, and visually representing said data.

Advantageously in the system of the invention, the means for calculation and data representation are adapted for carrying out the following steps:

b) calculating the variability on said $M$ intervals of a sequence of consecutive vectors $\{\delta_j\}_{j=1,\ldots,M-N}$ of $N$ components, according to the algorithm or transformation defined by the expression:

$$\delta_j = \left\{ \sum_{n=0}^{m} \binom{m}{n} (-1)^n \left( \langle X \rangle_{N_0,j\cdot\varsigma_0+K_0}^{-1} X_{j+n+k\cdot\varepsilon_0+J_0} - \varepsilon_1 \langle X \rangle_{N_1,j\cdot\varsigma_1+K_1}^{-1} \langle X \rangle_{N_2,j+n+k\cdot\varepsilon_2+K_2} \right) \right\}_{k=J_1,\ldots,J_1+N-1},$$

and the following notation:

$$\langle X \rangle_{L,l} = L^{-1} \sum_{h=0}^{L-1} X_{l+h} \text{ , with } \binom{m}{n} = \frac{m!}{n!(m-n)!},$$

where the following parameters are integers and their selection determine the final form of the mentioned transformation:

$$\{m, N, N_0, N_1, N_2, \varepsilon_0, \varepsilon_1, \varepsilon_2, \varsigma_0, \varsigma_1, J_0, J_1, K_0, K_1, K_2\},$$

where additionally:

- $m$ is a natural indicator representing the order of the discrete variation that is calculated;
- $N$ is the dimension or number of components of each vector $\delta_j$, where $N \geq 2$;
- $N_0, N_1,$ and $N_2$ indicate the number of values that are used for calculating the corresponding indicated local average in the general formula of the algorithm;
- $\varepsilon_0, \varepsilon_1, \varepsilon_2$ have binary values 0 or 1, and indicate if the corresponding elements are, respectively, fixed or mobile in the calculation of each of the components of the vector $\delta_j$;
- $\varsigma_0, \varsigma_1$ have binary values 0 or 1, and indicate if the local mean is, respectively, fixed or mobile;
- $J_0$ and $J_1$ indicate the delay or advance of the first element that is taken in the calculation on the basis of the indicator $j$;
- $K_0, K_1, K_2$ indicate the delay or advance of the first element that is taken in the corresponding local series for calculating the indicated local average;

wherein the position of the point indicated by the values of the components of each of the vectors $\delta_j$ is graphically

represented in two or more dimensions;

c) calculating the distance from the center of mass of the group $\{\delta_j\}_{j=1,...,M-N}$ to an origin of coordinates, as determined by:

$$\Phi_N = \left[\sum_{k=1}^{N-1}\left(\sum_{i;N}^{M-N+1}\delta_{i,k}\right)^2\right]^{1/2};$$

where $\delta_{i,k}$ is the component $k$ of the vector $\delta_i$, and the notation of the sum

$$\sum_{i;N}^{M-N+1}\delta_{i,k}$$

indicates N by N hops in the indicator $i$;

d) visually representing said distance from the center of mass in a system of coordinates, through the means of visual representation.

In a preferred embodiment of the invention, the means for calculation and visual representation are adapted for carrying out an additional step of comparing the useful data obtained with behavioral patterns associated with a vector function $A = \{a_j\}_{j=1,...,N}$, corresponding to the parameterization of a heart sequence, where the elements $a_j$ are fixed values or functions of one or more variables, and where an additional step of comparing the useful data obtained with said function A is performed according to the following steps:

a) calculating the general angle $\theta_i$, the cosine of which is determined by:

$$\cos(\theta_i) = \frac{A\cdot\delta_i}{\|A\|\cdot\|\delta_i\|},$$

where the symbol is the general norm of a vector in $N$ dimensions, such that:

$$\|A\| = \left(\sum_{j=1}^{N}a_j^2\right)^{1/2};$$

b) calculating the number of events $M'$ such that the angle $\theta_i$ is less than a predetermined tolerance $\varepsilon$, where $0 < \varepsilon < 1$, such that the function $A$ is explored in its space of existence in order to find said events in which $\theta_i < \varepsilon$;

c) calculating the coefficient $M'/M$;

d) visually representing the calculated data.

In a further preferred embodiment of the invention, the calculation of the series $\{\delta_j\}_{j=1,...,M-N}$ of consecutive vectors of $N$ dimensions or components is performed according to the following definitions of parameters:

$$\left\{ \begin{array}{l} m = 0 \\ N = 5 \\ N_0 = 5 \\ N_1 = 5 \\ N_2 = 5 \\ \varepsilon_0 = 1 \\ \varepsilon_1 = 1 \\ \varepsilon_2 = 0 \\ \varsigma_0 = 1 \\ \varsigma_1 = 1 \\ J_0 = 0 \\ J_1 = 0 \\ K_0 = 0 \\ K_1 = 0 \\ K_2 = 0 \end{array} \right\},$$

such that the definition of the variability on said M intervals is:

$$\delta_j = \left\{ X_{j+k} \langle X \rangle_{N,j}^{-1} - 1 \right\}_{k=0,\dots,N-1}.$$

In a further preferred embodiment of the invention, the means for calculation and data representation are adapted for carrying out the following steps:

a) calculating the number of events on the basis of the series $\{X_i\}_{i=1,\dots,M}$ with the specific definition of the vector $A = t\{(N+1)/2 - j\}_{j=1,\dots,N}$, where $N > 1$;
b) using the indicator $m_{S1}/M$, with $m_{S1} = M'$ calculated in the preceding point, for determining the existence of behavioral patterns associated with the vector function $A$;
c) visually representing the calculated data.

Alternatively, in a further preferred embodiment of the invention the means for calculation and data representation are adapted for carrying out the following steps:

a) calculating the number of events $M'$ on the basis of the series $\{X_i\}_{i=1,\dots,M}$ with the specific definition of the vector $A_N = t\{\sin(2\pi j)/N\}_{j=1,\dots,N}$, where $N$ can range from $N = 3$ to $N = 12$, corresponding to a sinusoidal modulation of the heart rhythm combined with the respiratory rhythm, where $t$ can have any value;
b) using the indicator $m_{S2}/M$, with $m_{S2} = M'$ calculated in the preceding point, for determining the existence of behavioral patterns associated with the vector function $A$;
c) visually representing the calculated data.

Alternatively, in a further preferred embodiment of the invention, the means for calculation and data representation are adapted for carrying out the following steps:

a) calculating the coefficient $M'/M$ on the basis of the series $\{X_i\}_{i=1,\dots,M}$ with the definition of the vector

$$A_N = t \left\{ -1, 1, \underset{N}{\underbrace{0, \dots, 0}} \right\}$$

, where $N$ can range from $N = 1$ to $N = 20$, corresponding to a compensated ectopic beat, and where t can have any value;
b) using the indicator $m_E/M$, with $m_E = M'$ calculated in the preceding step, for determining the existence of behavioral patterns associated with the vector function $A_N$;
c) visually representing the calculated data.

In a further preferred embodiment of the invention, the following steps are additionally performed:

a) calculating the coefficient $M'/M$ on the basis of the series $\{X_i\}_{i=1,...,M}$ with the specific definition of the vector

$$A_N = t\left\{N, \underbrace{-1, ..., -1}_{N}\right\}$$

, where $N$ can range from $N = 2$ to $N = 20$, corresponding to a regular paroxysmal tachycardia, and where t can have any value;

b) using the indicator $m_{TP}/M$, with $m_{TP} = M'$ calculated in the preceding point, for determining the existence of behavioral patterns associated with the vector function $A_N$;

c) visually representing the calculated data.

In a further preferred embodiment of the invention, the component of the heart "pQRSt" complex is the RR interval of an electrocardiogram.

In a further preferred embodiment of the invention, the recording of a number $M$ of consecutive time intervals $\{X_i\}_{i=1,...,M}$, corresponding to cycles of a component of a heart "pQRSt" complex is performed with a precision greater than 0.01% of the mean value of the cycle time.

In a further preferred embodiment of the invention, $0 < \varepsilon < 0.1$.

In a further preferred embodiment of the invention, t is 1 or -1.

## DESCRIPTION OF THE DRAWINGS

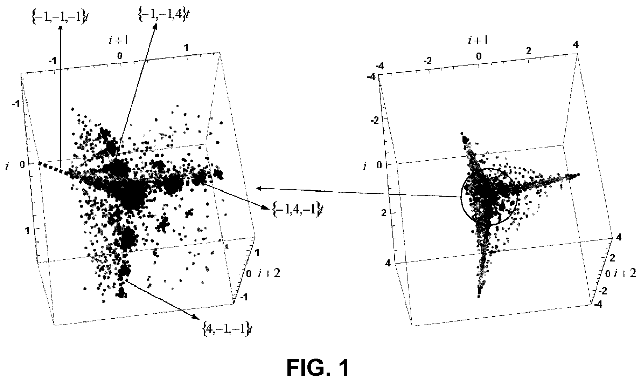

Figure 1. Representation of the distance of the vectors for a particular case of heart failure. Characteristics lines of this specific case and their mathematical expressions are shown. Furthermore, central regions of the graph have been enlarged with the areas of interest highlighted in order to see the details.

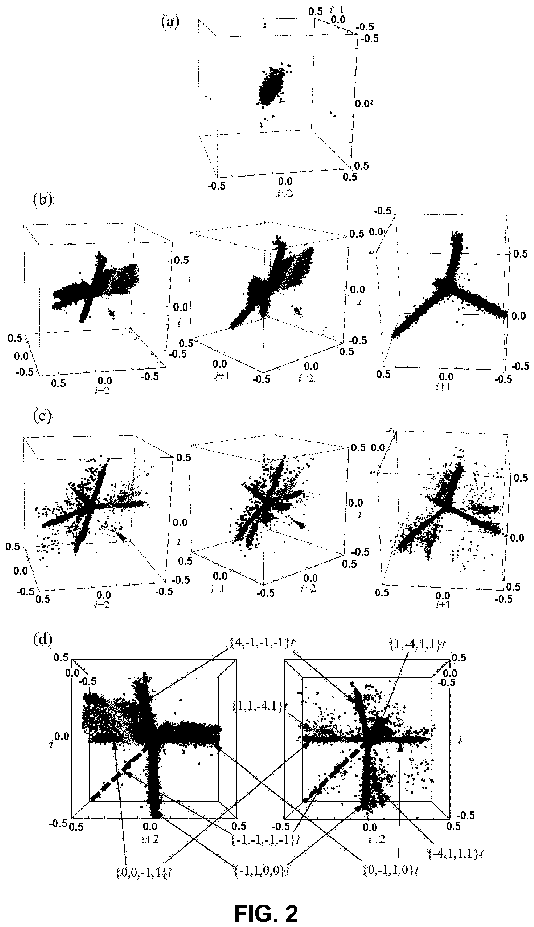

Figure 2. (a) Four-dimensional graph of the location of M-3 vectors $\{\Delta_i\}_{i=1,...,M-N+1}$ normalized with the overall mean of a normal HR (a healthy adult). (b, c) The same for two subjects with chronic heart failure (three viewing angles of each). The graphs located on the right in (b, c) show universal patterns when projected with the suitable angle. (d) Identification of the main lines of the heart failure in two different individuals with HF.

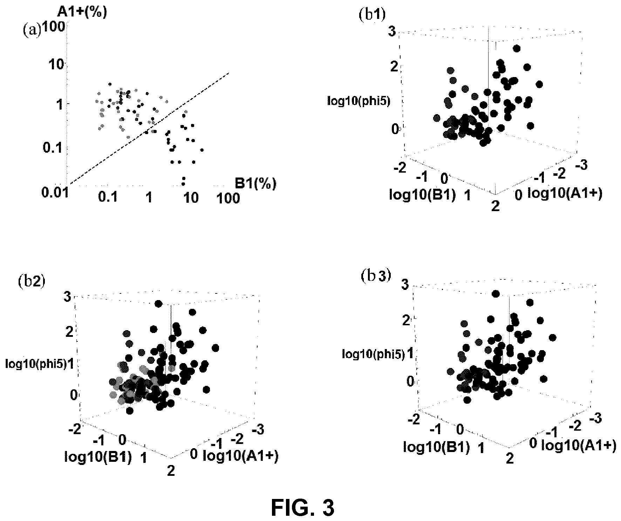

Figure 3. (a) The different regions of normal activity and of HF found in this study: NSR); HF (b1-b3) The different regions occupied by the four situations found in this study in the multivariable space $\{\log_{10}(A1), \log_{10}(B1), \log_{10}(\Phi_N)\}$. For greater clarity, the left panel provides three situations (NSR normal activity, myocardial infarction and HF), the central panel provides four situations (NSR normal activity, MI, SD and HF), and the right panel provides all the situations studied. N = 5 in all the results of this Figure.

## DETAILED DESCRIPTION OF THE INVENTION

A map of Poincaré is a graph consisting of the representation of a recurrence map or sequential path of the values having a certain variable in consecutive cycles. Specifically, a two-dimensional (2D) recurrence map is a planar projection where complex paths, with multidimensional characteristics (i.e., specific arrhythmic sequences) are overlapping and indistinguishable. This invention allows for a generalization of said recurrence maps. Among other possibilities, a normalized variability using a moving average of order N=5 can be formulated. This formulation corresponds with the following selection of the fifteen values defining the base algorithm of the methods implemented by the system of the invention:

$$\left\{ \begin{array}{l} m = 0 \\ N = 5 \\ N_0 = 5 \\ N_1 = 5 \\ N_2 = 5 \\ \varepsilon_0 = 1 \\ \varepsilon_1 = 1 \\ \varepsilon_2 = 0 \\ \varsigma_0 = 1 \\ \varsigma_1 = 1 \\ J_0 = 0 \\ J_1 = 0 \\ K_0 = 0 \\ K_1 = 0 \\ K_2 = 0 \end{array} \right\}.$$

By means of this selection, referred to as NL (Local Normalization), an expression of the sequential variability $\{\delta_j\}_{j=1,\dots,M-4}$, with $\delta_j = \left\{ X_{j+k} \langle X \rangle_{N,j}^{-1} - 1 \right\}_{k=0,\dots,4}$, $\langle X \rangle_{N,j} = \sum_{i=0}^{N-1} X_{j+i}$, is obtained and the representation thereof meets the aforementioned requirements for an ideal representation of the heart function in terms of compactness and integrity, and furthermore it reduces to a minimum exogenous and accidental influences as a result of the local normalization resulting from the values $\{X_i\}_{i=1,\dots,M}$. The proposed method can be applied to any of the components of the heart "pQRSt" complex, although this example focuses on the analysis of the main component, the series of RR intervals. Generally, by exploring the interval of order from N=2 to N=100, it can be seen that a quantification measurement of the overall variability, such as the norm or argument of the center of mass vector of the distribution of points defined by the vectors $\delta_j = \left\{ X_{j+k} \langle X \rangle_{N,j}^{-1} - 1 \right\}_{k=0,\dots,N-1} \equiv \left\{ \delta_{j,k} \right\}$, recurrently has a minimum for N=5 in the case of healthy individuals. This could be due to the fact that the average heart rate is about five times greater than the respiratory rate in the human species, which gives rise to a subharmonic of order five of the HRV, thereby maximizing overall compensation when N=5. If this hypothesis is correct, deviations from normality should optimally be distinguished using the order N=5.

Fortunately, N=5 gives rise to the most complete graph, with complete graphical representation possibilities, among all the possible orders. In fact, the expression $\delta_j = \left\{ X_{j+k} \langle X \rangle_{N,j}^{-1} - 1 \right\}_{k=0,\dots,N-1}$ allows the representation thereof in N-1 dimensions since the information contained in N-1 of the total of N elements provides all the information of the vector of N dimensions. That is because the value of any of the N elements can be obtained on the basis of the other N-1 since the sum of the N elements is always nil, by definition. The resulting four-dimensional sequential information vector, which can be graphically represented in 3D plus color, gives rise to a valuable descriptive and comparative tool from the clinical point of view. This graph allows the identification of sequences that originate universal patterns, the distribution and density of which provide an immediate and complete information about the state of cardiac function. Up to five universal sequences, which can particularly be seen in the graphical representation (see an example in Figure 1, with N=4, and other examples in Figure 2, with N=5 and another selection of parameters), have been identified using the method described, but they are not only present when N = 5: they can all be found in higher orders, and their general expressions for any order N have in fact been obtained.

The universality of these sequences has been verified in a series of HR databases, with 133 records clustered into in four basic situations with distinctive characteristics: (i) individuals in normal sinus rhythm (NSR) during normal activity, or at rest in the supine position while watching the film "Fantasia"; (ii) ischemic cardiopathy, specifically myocardial infarction (MI); (iii) heart failure (HF); and (iv) recovery from sudden death (SD). By applying the mentioned databases to it, the percentage of occurrence of each of the five sequences and the primary variability (the definition of which will be provided below) provide a univocal and specific set of measurements capable of assessing the state of the heart in the records included in the publically available databases that were used (see databases used below).

A Holter recorder (HR) is a set M of consecutive values corresponding to the RR intervals, which can be expressed as $\{X_j\}_{j=1,\ldots,M}$. It is easy to demonstrate that the general distance vector in N dimensions from any point $\{X_{j+k}\}_{k=0,\ldots,N-1}$ formed by N RR intervals to the line of identity (line of zero variability, Piskorski & Guzik 2012), defined by the identity vector $\{1,1,\ldots,1\}_N$, is defined as $D_j = \{X_{j+k} - \langle X \rangle_{N,j}\}_{k=0,\ldots,N-1}$. The sequence of RR intervals of a healthy heart "would dance" around the line of identity but would never be supported on it (even in the extreme situations of complete relaxation or extreme exercise, there is a variability mathematically different from zero, even though the RRs are apparently constant). The locally normalized expression of said general distance vector is precisely the expression

$$\delta_j \equiv \langle X \rangle_{N,j}^{-1} D_j = \left\{ \langle X \rangle_{N,j}^{-1} X_{j+k} - 1 \right\}_{k=0,\ldots,N-1}$$

of the selection of NL parameters, i.e., , where the basis for normali-

$$\langle X \rangle_{N,j} = \sum_{i=0}^{N-1} X_{j+i}$$

zation is the local mean . In Figure 1, a simple color code ranging between 0 and 1 (the Hue code of the Mathematica® 9.0 program) has been used as a fourth dimension.

Another selection of parameters that has been used for this study is the following:

$$\left\{ \begin{array}{l} m = 0 \\ N = 5 \\ N_0 = 5 \\ N_1 = M \\ N_2 = M \\ \varepsilon_0 = 1 \\ \varepsilon_1 = 1 \\ \varepsilon_2 = 0 \\ \varsigma_0 = 0 \\ \varsigma_1 = 0 \\ J_0 = 0 \\ J_1 = 0 \\ K_0 = 0 \\ K_1 = 0 \\ K_2 = 0 \end{array} \right\},$$

which gives rise to the following expression $\delta_j = \left\{ \langle X \rangle_M^{-1} X_{j+k} - \langle X \rangle_M^{-1} \langle X \rangle_{N,j} \right\}_{k=0,\ldots,N-1}$. This selection of parameters shall be referred to as NG (Overall Normalization), and the particular expression of $\delta_j$ for this selection will be referred to as $\Delta_j$. This expression has the following trivial relations with $D_j = \{X_{j+k} - \langle X \rangle_{N,j}\}_{k=0,\ldots,N-1}$ and with

$$\delta_j = \left\{ X_{j+k} \langle X \rangle_{N,j}^{-1} - 1 \right\}_{k=0,\ldots,N-1} :$$

$$\Delta_j = \langle X \rangle_M^{-1} D_j \text{, and } \Delta_j = \langle X \rangle_M^{-1} \langle X \rangle_{N,j} \delta_j.$$

Figure 2 shows the graphical representation of three HRs, analyzed with the proposed method, for N = 5: Figure 2 (a) represents a healthy subject, and Figure 2 (b, c) represents two patients with chronic heart failure. While in the healthy individual it gives rise to a dense and compact shape around the origin, individuals with HF show distinctive spatial lines that follow previously established sequences. In view of this figure, some immediate conclusions can be extracted. First, the lines shown are fundamentally straight. In some cases (for example, Figure 2 (b)), the space between two lines is joined by a characteristic plane, but there is a special viewing angle (angle of projection over the viewing plane) which systematically reduces the main straight lines and planes to just three in all cases [see Figure 2 (c)].

As would be expected, the normalized Poincaré sections of the variability of the NSR database (apparently non-pathological) are relatively centered and homogenously distributed around zero, showing a mathematical compensation (apparently) with certain randomization, i.e., a more or less spherical nucleus with a random, approximately Gaussian distribution. A more detailed observation shows that all the cases show a clear structure in the fourth dimension (color), along a specific direction. It is important to point out that this direction is exactly the same for all subjects, regardless the shape of the distribution of points representing the RR intervals. This fact points towards the existence of a subharmonic, probably related to the respiratory cycle, that reflects an automatism of the sympathetic-parasympathetic system. This effect is relatively rare or virtually absent in individuals with IM and is not found in individuals with HF and SD. The existence of an individual in the NSR database (around 12%) having the characteristics present in HF is quantified and discussed below, and it supports the fact that the presence of the sinus rhythm does not rule out the presence of a heart pathology.

The "Fantasia" database shows the same characteristics (Iyengar *et al.* 1996; Schimitt *et al.* 2007) as the individuals in the NSR database, and almost the same percentage of subjects with HF characteristics. Furthermore, elderly healthy subjects clearly show less variability than young healthy subjects.

Almost 70% of the subjects in the HF database (Baim *et al.* 1986), and many of the subjects studied in the SD database (Taddei *et al.* 1992) show the same distinctive lines mentioned above. The percentage of subjects with homogenously distributed point clouds is proportionally inverse when compared with the individuals in the NSR database: less than 20%. On the other hand, the repetition of the patterns and their extent comply with the universal characteristics of HRV. Finally, individuals in the SD database used in this study show variable patterns that are completely different and visibly more irregular than any other group. Some of them have the same lines as the individuals with HF, but most of them show a very complex and apparently chaotic structure.

As previously described in the preceding sections, the invention relates to a system for obtaining data associated with a heart rate variability (HRV) pattern, essentially comprising:

a1) means for measurement and data recording of an electrocardiogram, adapted for measuring and recording a number $M$ of consecutive time intervals $\{X_i\}_{i=1,...,M}$ corresponding to cycles of one or more components of a heart "pQRSt" complex of an electrocardiogram, with a precision equal to or greater than 10% of the mean value of the cycle time, and $M$ being greater than 2;

a2) means for calculation and visual representation, adapted for performing calculations on the data recorded by the means for measuring and data recording, and visually representing said data;

Advantageously, the means for calculation and data representation are adapted for carrying out the following steps:

b) calculating the variability on said $M$ intervals of a sequence of consecutive vectors $\{\delta_j\}_{j=1,...,M-N}$ of $N$ components, according to the algorithm or transformation defined by the expression:

$$\delta_j = \left\{ \sum_{n=0}^{m} \binom{m}{n} (-1)^n \left( \langle X \rangle_{N_0, j \cdot \varsigma_0 + K_0}^{-1} X_{j+n+k \cdot \varepsilon_0 + J_0} - \varepsilon_1 \langle X \rangle_{N_1, j \cdot \varsigma_1 + K_1}^{-1} \langle X \rangle_{N_2, j+n+k \cdot \varepsilon_2 + K_2} \right) \right\}_{k=J_1,...,J_1+N-1},$$

and the following notation:

$$\langle X \rangle_{L,l} = L^{-1} \sum_{h=0}^{L-1} X_{l+h}, \text{ with } \binom{m}{n} = \frac{m!}{n!(m-n)!},$$

where the following parameters are integers and their selection determine the final form of the mentioned transformation:

$$\{m, N, N_0, N_1, N_2, \varepsilon_0, \varepsilon_1, \varepsilon_2, \varsigma_0, \varsigma_1, J_0, J_1, K_0, K_1, K_2\},$$

where additionally:

-   $m$ is a natural indicator representing the order of the discrete variation that is calculated;

- $N$ is the dimension or number of components of each vector $\delta_j$, where $N \geq 2$;
- $N_0, N_1$, and $N_2$ indicate the number of values that are used for calculating the corresponding indicated local average in the general formula of the algorithm;
- $\varepsilon_0, \varepsilon_1, \varepsilon_2$ have binary values 0 or 1, and indicate if the corresponding elements are, respectively, fixed or mobile in the calculation of each of the components of the vector $\delta_j$;
- $\varsigma_0, \varsigma_1$ have binary values 0 or 1, and indicate if the local mean is, respectively, fixed or mobile;
- $J_0$ and $J_1$ indicate the delay or advance of the first element that is taken in the calculation on the basis of the indicator $j$;
- $K_0, K_1, K_2$ indicate the delay or advance of the first element that is taken in the corresponding local series for calculating the indicated local average;

wherein the position of the point indicated by the values of the components of each of the vectors $\delta_j$ is graphically represented in two or more dimensions;

c) calculating the distance from the center of mass of the group $\{\delta_j\}_{j=1,\ldots,M-N}$ to an origin of coordinates, as determined by:

$$\Phi_N = \left[ \sum_{k=1}^{N-1} \left( \sum_{i;N}^{M-N+1} \delta_{i,k} \right)^2 \right]^{1/2} ;$$

where $\delta_{i,k}$ is the component $k$ of the vector $\delta_i$, and the notation of the sum

$$\sum_{i;N}^{M-N+1} \delta_{i,k}$$

indicates N by N hops in the indicator $i$;

d) visually representing said distance from the center of mass in a system of coordinates, through the means of visual representation.

A first characteristic of the graphical representation proposed in this invention with both the NL and the NG selection ($\delta_j$ or $\Delta_j$) is that it is centered around the origin by definition, such that the density of points in different areas can be considered a specific identifying signature of variability. Therefore, the norm of the normalized vector defining the center of mass of the graphical representation would be a primary measurement of the variability for an n-th subharmonic order, and of its overall degree of compensation (for example, in an HR of 24 hours for a circadian cycle). However, experience has shown that the entire record of vectors $\Delta_j$ gives rise to an inadequate compensation due to the inherent nature of the Poincaré representation: it can be seen that the same arrhythmic RR interval $X_j$ appears as a common component in N - 1 vectors $\Delta_{j-k}$ around the line of identity, which often produces an apparent overall compensation. To avoid this, a subset of all the series can be extracted by taking the indicator $j$ in hops of N elements (i.e., $j = \{1, 1 + N, 1 + 2N, \ldots\}$), where each $X_j$ appears only once. The distance from the center of mass of this subgroup to the origin, the graphical representation of which is virtually indistinguishable from that of the entire series except for the different densities thereof, is determined by:

$$\Phi_N = \left[ \sum_{k=1}^{N-1} \left( \sum_{i;N}^{M-N+1} \Delta_{i,k} \right)^2 \right]^{1/2} ,$$

where $\Delta_{i,k}$ is the component $k$ of the vector $\Delta_i$, and the notation of the sum $\sum_{i;N}^{M-N+1} \Delta_{i,k}$ indicates N by N hops in the indicator $i$. This coefficient, referred to as primary variability (PV), can be represented for each individual as a function of N. Experience shows that $\Phi_N$ is about 10 to 100 times greater than the distance to the origin of the center of mass of the complete original set, which greatly amplifies the significance of $\Phi_N$ as it has been defined. It also depends on the number of beats in the series. In general, the minimum variability corresponds to NRS (Fantasia) individuals, closely followed by the variabilities of individuals with MI. The maximum variability corresponds to SD and HF, the distribution thereof being fairly similar. The variability of subjects in the NSR database with normal activity is at intermediate values. $\Phi_N$ is shown as a new quantitative measurement with extraordinary capacity to differentiate among patients with different heart function disturbances, in combination with the proposed graphical representation and arrhythmic structures derived from it.

A first classification of the different patterns that can be clustered into universal lines or sequences is provided below. On the other hand, it will be demonstrated that the density of points along those lines provides a valuable measurement of the state of the heart function. In fact, when the method implemented by the system of the invention is applied to the databases used, which are freely available, it gives rise to results with a high descriptive specificity for each group. As a first approach, the present invention is limited to the identification of the simplest primary arrhythmic sequences, which can be expressed in the form of a line, defined by the vector

$$A_N = t\{a_1, a_2, ..., a_N\},$$

parameterized by an arbitrary variable $t$. Logically, the position vector of a real point (beat) on a specific arrhythmic line may correspond to any positive real value $t$. This reflects the intrinsic capacity of the described method: an arrhythmic line (or anomaly), which can be mathematically and universally expressed, brings together all the arrhythmias of the same nature, regardless of the heart rate and amplitude of variability. Several primary sequences that will be described below have been deciphered, and the density of points along the corresponding lines (sequences) has been calculated. These results do not exclude the existence of other more complex sequences, with specific associated characteristics that will be determined in future studies.

An immediate way to evaluate the density of points corresponding to a specific sequence is to quantify their presence in % through the entire total record of the series of RR. Given that the presence of a specific sequence is obviously not an exact or uniform amount in all the situations or in all individuals, it is necessary to use statistical means to determine the higher or lower presence of said sequence in a given heart condition. A convenient way to represent the distribution of the presence of a sequence in a certain condition is to determine the value of $F_i = i/M_B$ versus $y_i$ for the corresponding database, $y_i$ being the percentage of presence of the particular sequence $A_N = t\{a_1, a_2, ..., a_N\}$ of an individual, $i$ being the range of said individual in particular, based on his or her score $y_i$, and $M_B$ being the total number of individuals in the database. This would be determined for each sequence and analyzed in combination for each situation.

Arrhythmia A1: a healthy individual with NSR must show an intrinsic capacity for responding to any demand of the organism, by means of regular accelerations and decelerations of the heart rate directed by the sympathetic/parasympathetic balance. This capacity must be reflected in the appearance of the simplest form of HRV, which can be expressed as a linear ramp:

$$A1 = t\{(N+1)/2 - j\}_{j=1,...,N}$$

where the positive or negative sign of $t$ is applied to the acceleration or deceleration of the heart rate, respectively. For example, for N = 4, with an accelerated heart rate, it would be formulated as $A1_4^+ = t\{1.5, 0.5, -0.5, 1.5\}$, with $t > 0$; for N = 5 and a decelerated heart rate, it would be $A1_5^- = t\{-2, -1, 0, 1, 2\}$, with $t > 0$, etc., where the higher values of $t$ indicate a more abrupt rise or fall, or a more pronounced ramp, of the heart rate, without changes in the functional structure of the variability.

This is the dominant form of heart rate variability (HRV) in normal individuals in the MIT-BIH NSR database, as shown in Table 1. In fact, this is reflected in a slightly ellipsoid shape around the origin in the direction of the line of $A1_5$ of any four-dimensional graph of a Poincaré map of the fifth order, which represents the HRV of a normal individual with NSR, as has been proposed herein [see Figure 2 (a)].

Even more importantly, as the presence of this form of HRV decreases, other forms of pathological arrhythmias, which will be described below, increase by about the same proportion. This finding fundamentally points towards a basic organic fact, i.e., this arrhythmia is actually the basic degree of freedom of the HRV and reflects the adaptive capacity of a healthy organism. If a pathological situation depresses or limits this degree of freedom, other forms of HRV will manifest to compensate for this deficiency. It is important to point out that these alternative forms are not arbitrary, particularly in the individuals included in the HF database. In fact, they can be considered universal. Accordingly, the specific form of these alternative HRV patterns must be linked with the specific condition of an organism, which opens up the door to new forms of rapid, non-invasive diagnosis.

Arrhythmia B1 (compensated ectopic beat): The four-dimensional graph of HR belonging to individuals with HF generally have three lines (Figure 2 (d)) which can be easily identified as (see Figure 2 (d)):

$$B1_{5,1} = t\{-1,1,0,0\}, \ B1_{5,2} = t\{0,-1,1,0\}, \ B1_{5,3} = t\{0,0,-1,1\}$$

First of all, these three sequences actually pertain to a single class of the following type

$$t\{...,0,0,-1,1,0,0,...\}$$

Second of all, the mean value of the four components is zero for any N>2, and these sequences can therefore be considered "compensated." This means that the final point of the Poincaré section of the n-th order, rests approximately on the line of identity. In other words, the arrhythmic sequence can also be considered "closed" or "concluded" since the last interval lasts for the same time as the local mean. The fact that the number of intervals with zero variability surrounding the succession {-1, 1} in most cases is greater than two (i.e., the line {1, 0, 0, -1} does not clearly appear) should be pointed out. It can therefore be concluded that there is a specific sequence described by the following line:

$$B1 = t\{...,0,0,-1,1,0,0,...\},$$

having clear ubiquity: this type of arrhythmia is definitively characteristic and clearly dominant in individuals with HF, with a mean of presence around an order of magnitude greater in HF than in SD or MI, despite the fact that it is also dominant in these subjects. While the primary variabilities (PV) of individuals in the HF and SD databases are large and very similar, what really distinguishes individuals with HF from those recovering from SD is the much higher presence in the former of arrhythmia B1. These arrhythmias correspond to compensated isolated ectopic beats; the present invention does not seek to identify their specific cardiac origin, whether supraventricular or ventricular, since the nature thereof may be associated with another characteristic that has not yet been studied.

As can be seen, this analysis can provide a new basis for elaborating a general classification based on the inherent and strongly compensated nature of these arrhythmias and their capacity to be reduced to a single universally expressible structure. On the other hand, these are arrhythmias that are relatively rare or absent in individuals in the NSR database. In fact, ectopic beats may occur in the recordings of normal individuals, but they are relatively rare. However, arrhythmias of this type are more present in the recordings of awake individuals at rest and in the supine position ("Fantasia" record mentioned below) than in normal individuals during normal activity. Nevertheless, the prevalence of arrhythmia B1 in HF may require a future review of the diagnostic value (Frolkis *et al.* 2003; and related references) of the presence of ectopic beats in combination with the capacity to effortlessly adapt to the demands of normal activity (adaptability associated with the presence of arrhythmias A1). Table 1 shows the strong inverse correlation between the presence of compensated ectopic beats (arrhythmias B1) and arrhythmias A1 ($\pm$). In addition to Table 1, the opposing presence of arrhythmias A1 and B1 is clearly illustrated in Figure 3.

Arrhythmia B2 (regular paroxysmal tachycardia): The graphical representation shows the sequence described by [see Figure 1 (a, b)]:

$$B2_{5,1} = t\{4,-1,-1,-1\}$$

In many cases an additional line appears [see Figure 1 (c, d)]:

$$B2_{5,2} = t\{-1,-1,-1,-1\}$$

First of all, the occurrence of these sequences introduces an additional source of randomization upon being combined with A1. This provides an additional adaptive capacity which, on the other hand, may be very limited (it would look like a more intense failure) as the presence of arrhythmias A1 decreases (normal adaptability) in individuals with heart pathologies. Second of all, none of these sequences is compensated. This means that values of N of a higher order must be considered in order to find compensated or finished sequences. In this case, both $B2_{5,1}$ and $B2_{5,2}$ are actually part of the compensated sequence:

$$B2 = t\{4,-1,-1,-1,-1\}$$

which actually pertains to a Poincaré section of the sixth order (i.e., N = 6). It can be seen that compensated sequences are described by lines of the following type:

$$B2_N = t\{(N-2), -1, \ldots, -1\},$$

for example:

$$B2_6 = t\{4, -1, -1, -1, -1\}$$

$$B2_7 = t\{5, -1, -1, -1, -1, -1\}$$

$$B2_8 = t\{6, -1, -1, -1, -1, -1, -1\}$$

$$B2_9 = t\{7, -1, -1, -1, -1, -1, -1, -1\}$$

etc. All these sequences are almost as present as arrhythmia B1 in HF, although the presence of this type of arrhythmia drops as N increases. This sequence is hardly identifiable if N increases above 10. Actually, the arrhythmia with the greatest presence is $B2_6$, and that is why this type of arrhythmia is generically referred to as $B2$ instead of $B2_6$. The analysis of the presence of this arrhythmia in HF demonstrates that it is as omnipresent as B1, with a different dominance of one over the other, depending on the individual. This sequence is compatible with a regular paroxysmal tachycardia after a pause, which can be attributed to an atrioventricular block, which in some cases gives rise to Stokes-Adams syndrome. Its frequency of presentation in each situation is shown in Table 1. Like arrhythmia B1, arrhythmia B2 is characteristic of HF. It is important to take into account that this arrhythmia represents a noticeable pause, followed by a proportional rapid-rate series to compensate for the pause.

Arrhythmia B3 (regular paroxysmal tachycardia II): several sequences that are alternatives to B2 can be identified, such as:

$$B3_{6,1} = t\{-1, 4, -1, -1, -1\}$$

$$B3_{6,2} = t\{-1, -1, 4, -1, -1\}$$

$$B3_{6,3} = t\{-1, -1, -1, 4, -1\}$$

$$B3_{6,4} = t\{-1, -1, -1, -1, 4\},$$

or:

$$B3_{7,1} = t\{-1, 5, -1, -1, -1, -1\}$$

$$B3_{7,2} = t\{-1, -1, 5, -1, -1, -1\}$$

$$B3_{7,3} = t\{-1, -1, -1, 5, -1, -1\}$$

$$B3_{7,4} = t\{-1, -1, -1, -1, 5, -1\}$$

$$B3_{7,4} = t\{-1,-1,-1,-1,-1,5\}$$

etc., which can be expressed for a general indicator N as :

$$B3_{N,i} = t\{-1,\ldots,-1, N-2 \,(\text{at position } i), -1,\ldots,-1\}.$$

For a given N, all the sequences of B3 with different indicators *i* have the same presence, but said presence is significantly lower than B2. This sequence could be similar to intermediate situations by joining two consecutive sequences corresponding to regular paroxysmal tachycardia with relative pauses like in arrhythmia B2. It is surprising that this arrhythmia is less characteristic of HF; actually, it is as present in HF as it is in SD, with an almost identical probability distribution. On the other hand, it is significantly more present in NSR than in the individuals with IM and in the individuals in the NSR ("Fantasia") database.

In some cases, a peculiar sequence appears as "shadows" of arrhythmia B2 [see Figure 2 (d), for example], which can be identified as lines:

$$\overline{B}3_{6,1} = t\{-4,1,1,1,1\}$$

$$\overline{B}3_{6,2} = t\{1,-4,1,1,1\}$$

$$\overline{B}3_{6,3} = t\{1,1,-4,1,1\}$$

which can generally be written as

$$\overline{B}3_{N,i} = t\{1,\ldots,1, -(N-2)(\text{at position } i), 1,\ldots,1\},$$

a somewhat complementary sequence of B3$_{N-2, i}$. A quantitative analysis of this type of arrhythmia is not provided given its complexity.

Arrhythmia A2 (relating to breathing): This is a relatively present sequence, though much less so than arrhythmia B1 or B2 in HF or SD, which appears as the dominant form of sub-arrhythmia in normal or asymptomatic individuals. This compensated sequence can be described by the following line:

$$A2_N = t\{\sin(2\pi \cdot j)/N\}_{j=1,\ldots,N},$$

representing a sinusoidal modulation of the heart rate along a range of N beats. This type of arrhythmia with mathematical compensation must also reflect a physiological compensation. Given that it is statistically more frequent with N = 5 than with any other order, it may be concluded that it is related to the mean respiratory rate in the human species. Whether or not it is more frequent during sleep than during normal activity will be the object of future studies. Its presence in the records is analyzed in Table 1. It is relatively dominant in NRS with respect to the pathological records, therefore it is significantly less frequent in individuals with HF and SD. It can be deduced that this arrhythmia is, like A1, characteristically non-pathological. In other words, its presence is compatible with a good state of heart health.

The combination of the densities of each identified arrhythmia may constitute a valuable characteristic signature of a specific situation, thereby opening up the pathway to new diagnostic examinations, the meaning of which is out of reach of this example of application of the proposed invention. A fundamental finding is the inverse relation between the presence of certain types of arrhythmia, those which may be considered to be indicative of "health", and those which may be referred to as "pathological". Specifically, in the analysis of the public databases used, arrhythmia A1 and arrhythmia B1 are antagonistic: in fact, the relative presence of one with respect to the other is reversed when going from a situation of normality to a pathological state. Figure 3 shows a universal map in which the presence of both arrhythmias in individuals in the NSR and HF databases is analyzed. A very clear difference can be seen between NSR and HF, based on the presence of A1 and B1.

Furthermore, the value of the primary variability $\Phi_N$ completes the set of characteristic variables for providing the distinctive, characteristic seal of each situation: it must be noted in Table 1 that the combinations of the presences of each arrhythmia and the PV makes up a unique and highly differentiated signature of each situation. The difference between MI and SD is $\Phi_N$, small for MI and large for SD. The multivariable probability density function for each situation in the space of variables {$\log_{10}(A1)$,$\log_{10}(B1)$,$\log_{10}(\Phi_N)$} would be determined by the density of the points where, in the space, it would correspond to each situation, as can be seen in Figure 3.

The clinical value of this representation could be extremely significant. In fact, the therapeutic effect of drugs with a specific cardiovascular action, targeting objectives such as sympathetic/parasympathetic axis (adrenergic beta-blockers), perhaps the neurohormonal rennin-angiotensin-aldosterone axis (IECAs, ARA II, etc.), etc., could give rise to the modification of the relative presence of each arrhythmia and displace the location of the corresponding graph in the direction of the NSR region. The potential prognostic value of the results obtained when applying the method implemented by the system of the present invention to HR is obvious. The observation of the four-dimensional graphical representation of HR allows the easy and immediate identification of disturbances in normality in healthy people.

Future clinical studies will expand the depth of knowledge acquired with the analysis proposed in this invention, such as the identification of new characteristics and new general arrhythmic patterns, relating to other pathologies and situations, not necessarily of a cardiac origin, for example, diabetes, hypertension, hypothyroidism, or even psychic disturbances.

Table 1

| Arrhythmia | NSR(normal activity) | "Fantasia" | MI | SD | HF |
|---|---|---|---|---|---|
| A1+ | 1.21849 | 0.841977 | 0.682145 | 0.355535 | 0.23905 |
| A1- | 0.725413 | 0.657101 | 0.502548 | 0.264764 | 0.188382 |
| A2+ | 0.417186 | 0.576313 | 0.320071 | 0.33573 | 0.158211 |
| A2- | 0.630867 | 0.58272 | 0.379129 | 0.284021 | 0.175146 |
| B1 | 0.354003 | 0.568972 | 1.7985 | 1.48564 | 5.59225 |
| B2 | 0.431622 | 0.32933 | 0.339216 | 0.493797 | 0.772125 |
| $\Phi_5$ | 10.1739 | 2.4295 | 1.7567 | 41.2053 | 48.9619 |

In conclusion, the present invention proposes a quantitative system which does not only provide a universal representation of heart rate variability, but also a clinical tool that is potentially useful in evaluating heart function, risks, and probably other related health issues. In the present invention, among the many different universal sequence types that probably exist, some patterns that stand out have been described mathematically with a relatively simple structure, identifying five general types of arrhythmias. The databases used allowed identifying the characteristic signatures of individuals included in the NSR, MI, HF, and SD databases, by relating these basic types of arrhythmias with the different situations of heart function. As a fundamental result, it has been quantitatively demonstrated that two of these arrhythmias are characteristic of the state of health, whereas the other three are pathological. Their relative presence in an individual may eventually be related to specific situations, with the growing clinical evidence provided by this methodology which will be built on in the future. Furthermore, the temporal evolution of arrhythmic structures in a patient, which are visible with the application of the methodology of the invention, can provide very valuable information about the state and/or clinical evolution of said patient. It is considered a methodology that is easy to apply, and the potential of its results in non-invasive clinical diagnosis and prognosis could be vital.

**Claims**

1. System for obtaining data associated with a heart rate variability (HRV) pattern, comprising:

   a1) means for measurement and data recording of an electrocardiogram, adapted for measuring and recording a number $M$ of consecutive time intervals $\{X_i\}_{i=1,\ldots,M}$ corresponding to cycles of one or more components of a heart "pQRSt" complex of an electrocardiogram, with a precision equal to or greater than 10% of the mean value of the cycle time, and $M$ being greater than 2;
   a2) means for calculation and visual representation, adapted for performing calculations on the data recorded by the means for measuring and data recording, and visually representing said data;
   **characterized in that** the means for calculation and data representation are adapted for carrying out the following steps:

b) calculating the variability on said $M$ intervals of a sequence of consecutive vectors $\{\delta_j\}_{j=1,\ldots,M-N}$ of $N$ components, according to the algorithm or transformation defined by the expression:

$$\delta_j = \left\{ \sum_{n=0}^{m} \binom{m}{n} (-1)^n \left( \langle X \rangle_{N_0, j \cdot \varsigma_0 + K_0}^{-1} X_{j+n+k \cdot \varepsilon_0 + J_0} - \varepsilon_1 \langle X \rangle_{N_1, j \cdot \varsigma_1 + K_1}^{-1} \langle X \rangle_{N_2, j+n+k \cdot \varepsilon_2 + K_2} \right) \right\}_{k=J_1,\ldots,J_1+N-1},$$

and the following notation:

$$\langle X \rangle_{L,l} = L^{-1} \sum_{h=0}^{L-1} X_{l+h}, \text{ with } \binom{m}{n} = \frac{m!}{n!(m-n)!},$$

where the following parameters are integers and their selection determine the final form of the mentioned transformation:

$$\{m, N, N_0, N_1, N_2, \varepsilon_0, \varepsilon_1, \varepsilon_2, \varsigma_0, \varsigma_1, J_0, J_1, K_0, K_1, K_2\},$$

where additionally:

- $m$ is a natural indicator representing the order of the discrete variation that is calculated;
- $N$ is the dimension or number of components of each vector $\delta_j$, where $N \geq 2$;
- $N_0, N_1,$ and $N_2$ indicate the number of values that are used for calculating the corresponding indicated local average in the general formula of the algorithm;
- $\varepsilon_0, \varepsilon_1, \varepsilon_2$ have binary values 0 or 1, and indicate if the corresponding elements are, respectively, fixed or mobile in the calculation of each of the components of the vector $\delta_j$;
- $\varsigma_0, \varsigma_1$ have binary values 0 or 1, and indicate if the local mean is, respectively, fixed or mobile;
- $J_0$ and $J_1$ indicate the delay or advance of the first element that is taken in the calculation on the basis of the indicator $j$;
- $K_0, K_1, K_2$ indicate the delay or advance of the first element that is taken in the corresponding local series for calculating the indicated local average;

wherein the position of the point indicated by the values of the components of each of the vectors $\delta_j$ is graphically represented in two or more dimensions;

c) calculating the distance from the center of mass of the group $\{\delta_j\}_{j=1,\ldots,M-N}$ to an origin of coordinates, as determined by:

$$\Phi_N = \left[ \sum_{k=1}^{N-1} \left( \sum_{i:N}^{M-N+1} \delta_{i,k} \right)^2 \right]^{1/2};$$

where $\delta_{i,k}$ is the component $k$ of the vector $\delta_j$, and the notation of the sum $\sum_{i:N}^{M-N+1} \delta_{i,k}$ indicates N by N hops in the indicator $i$;

d) visually representing said distance from the center of mass in a system of coordinates, through the means of visual representation.

2. System according to claim 1, wherein the means for calculation and visual representation are adapted for carrying out an additional step of comparing the useful data obtained with behavioral patterns associated with a vector function $A = \{a_j\}_{j=1,\ldots,N}$, corresponding to the parameterization of a heart sequence, where the elements $a_j$ are fixed values or functions of one or more variables, and where an additional step of comparing the useful data obtained with said function A is performed according to the following steps:

a) calculating the general angle $\theta_i$, the cosine of which is determined by:

$$\cos(\theta_i) = \frac{A \cdot \delta_i}{\|A\| \cdot \|\delta_i\|},$$

where the symbol is the general norm of a vector in N dimensions, such that:

$$\|A\| = \left( \sum_{j=1}^{N} a_j^2 \right)^{1/2};$$

b) calculating the number of events $M'$ such that the angle $\theta_i$ is less than a predetermined tolerance $\varepsilon$, where $0 < \varepsilon < 1$ , such that the function $A$ is explored in its space of existence in order to find said events in which $\theta_i < \varepsilon$;
c) calculating the coefficient $M'/M$;
d) visually representing the calculated data.

3. System according to the preceding claim, wherein the calculation of the series $\{\delta_j\}_{j=1,\ldots,M-N}$ of consecutive vectors of $N$ dimensions or components is performed according to the following definitions of parameters:

$$\begin{cases} m = 0 \\ N = 5 \\ N_0 = 5 \\ N_1 = 5 \\ N_2 = 5 \\ \varepsilon_0 = 1 \\ \varepsilon_1 = 1 \\ \varepsilon_2 = 0 \\ \varsigma_0 = 1 \\ \varsigma_1 = 1 \\ J_0 = 0 \\ J_1 = 0 \\ K_0 = 0 \\ K_1 = 0 \\ K_2 = 0 \end{cases},$$

such that the definition of the variability on said M intervals is:

$$\delta_j = \left\{ X_{j+k} \langle X \rangle_{N,j}^{-1} - 1 \right\}_{k=0,\ldots,N-1}.$$

4. System according to the preceding claim, wherein the means for calculation and data representation are adapted for carrying out the following steps:

a) calculating the number of events on the basis of the series $\{X_i\}_{i=1,\ldots,M}$ with the specific definition of the vector $A = t\{(N + 1)/2 - j\}_{j=1,\ldots,N}$, where $N > 1$;

b) using the indicator $m_{S1}$ / $M$, with $m_{S1}$ = $M'$ calculated in the preceding point, for determining the existence of behavioral patterns associated with the vector function $A$;

c) visually representing the calculated data.

5. System according to claim 3, wherein the means for calculation and data representation are adapted for carrying out the following steps:

a) calculating the number of events $M'$ on the basis of the series $\{X_i\}_{i=1,...,M}$ with the specific definition of the vector $A_N = t\{\sin(2\pi j)/N\}_{j=1,...,N}$, where $N$ can range from $N = 3$ to $N = 12$, corresponding to a sinusoidal modulation of the heart rhythm combined with the respiratory rhythm, where $t$ can have any value;

b) using the indicator $m_{S2}$ / $M$, with $m_{S2}$ = $M'$ calculated in the preceding point, for determining the existence of behavioral patterns associated with the vector function $A$;

c) visually representing the calculated data.

6. System according to claim 3, wherein the means for calculation and data representation are adapted for carrying out the following steps:

a) calculating the coefficient $M'/M$ on the basis of the series $\{X_i\}_{i=1,...,M}$ with the definition of the vector

$$A_N = t\left\{-1, 1, \underset{N}{\underline{0, ..., 0}}\right\}$$

, where $N$ can range from $N = 1$ to $N = 20$, corresponding to a compensated ectopic beat, and where t can have any value;

b) using the indicator $m_E$ / $M$, with $m_E$ = $M'$ calculated in the preceding step, for determining the existence of behavioral patterns associated with the vector function $A_N$;

c) visually representing the calculated data.

7. System according to claim 3, wherein the following steps are additionally performed:

a) calculating the coefficient $M'/M$ on the basis of the series $\{X_i\}_{i=1,...,M}$ with the specific definition of the vector

$$A_N = t\left\{N, \underset{N}{\underline{-1, ..., -1}}\right\}$$

, where $N$ can range from $N = 2$ to $N = 20$, corresponding to a regular paroxysmal tachycardia, and where t can have any value;

b) using the indicator $m_{TP}$ / $M$, with $m_{TP}$ = $M'$ calculated in the preceding point, for determining the existence of behavioral patterns associated with the vector function $A_N$;

c) visually representing the calculated data.

8. System according to any of the preceding claims, wherein the component of the heart "pQRSt" complex is the RR interval of an electrocardiogram.

9. System according to any of the preceding claims, wherein the recording of a number $M$ of consecutive time intervals $\{X_i\}_{i=1,...,M}$, corresponding to cycles of a component of a heart "pQRSt" complex is performed with a precision greater than 0.01% of the mean value of the cycle time.

10. System according to claim 3, wherein $0 < \varepsilon < 0.1$.

11. System according to any of claims 6-8, wherein t is 1 or -1.

**Patentansprüche**

1. System zum Gewinnen von Daten im Zusammenhang mit einem Herzfrequenzvariabilitätsmuster (heart rate variability, HRV), umfassend:

a1) Mittel zur Messung und Datenaufzeichnung eines Elektrokardiogramms, die zum Messen und Aufzeichnen einer Anzahl $M$ von aufeinanderfolgenden Zeitintervallen $\{X_i\}_{i=1,...,M}$ geeignet ist, was Zyklen einer oder mehrerer Komponenten eines Herz-"pQRSt"-Komplexes eines Elektrokardiogramms mit einer Genauigkeit von mindestens 10 % des Mittelwerts der Zykluszeit entspricht, wobei $M$ größer als 2 ist;

a2) Mittel zur Berechnung und visuellen Darstellung, die geeignet sind, Berechnungen an den durch die Mittel zur Messung und Datenaufzeichnung aufgezeichneten Daten durchzuführen und diese Daten visuell darzustellen;

**dadurch gekennzeichnet, dass** die Mittel zur Berechnung und Datendarstellung zum Durchführen der folgenden Schritte geeignet sind:

b) Berechnen der Variabilität an den $M$ Intervallen einer Folge aufeinanderfolgender Vektoren $\{\delta_J\}_{J=1,\ldots,M-N}$ von $N$ Komponenten gemäß dem Algorithmus oder der Transformation, der/die durch den Ausdruck definiert ist:

$$\delta_j = \left\{ \sum_{n=0}^{m} \binom{m}{n} (-1)^n \left( \langle X \rangle_{N_0,\, j\cdot\varsigma_0+K_0}^{-1} X_{j+n+k\cdot\varepsilon_0+J_0} - \varepsilon_1 \langle X \rangle_{N_1,\, j\cdot\varsigma_1+K_1}^{-1} \langle X \rangle_{N_2,\, j+n+k\cdot\varepsilon_2+K_2} \right) \right\}_{k=J_1,\ldots,J_1+N-1},$$

und der folgenden Schreibweise:

$$\langle X \rangle_{L,l} = L^{-1} \sum_{h=0}^{L-1} X_{l+h}, \quad \text{wobei} \quad \binom{m}{n} = \frac{m!}{n!(m-n)!},$$

wobei die folgenden Parameter ganze Zahlen sind und ihre Auswahl die endgültige Form der erwähnten Transformation bestimmt:

$$\{ m, N, N_0, N_1, N_2, \varepsilon_0, \varepsilon_1, \varepsilon_2, \varsigma_0, \varsigma_1, J_0, J_1, K_0, K_1, K_2 \},$$

wobei zusätzlich:

- $M$ ein natürlicher Indikator ist, der die Reihenfolge der berechneten diskreten Variation darstellt;
- $N$ die Dimension oder Anzahl der Komponenten jedes Vektors $\delta_J$ ist, wobei $N \geq 2$;
- $N_0$, $N_1$ und $N_2$ die Anzahl der Werte angeben, die zum Berechnen des entsprechenden angegebenen lokalen Durchschnitts in der allgemeinen Formel des Algorithmus verwendet werden;
- $\varepsilon_0$, $\varepsilon_1$, $\varepsilon_2$ Binärwerte 0 oder 1 aufweisen und angeben, ob die entsprechenden Elemente bei der Berechnung jeder der Komponenten des Vektors $\delta_J$ fest oder mobil sind;
- $\varsigma_0$, $\varsigma_1$ Binärwerte 0 oder 1 aufweisen und angeben, ob der lokale Mittelwert fest bzw. mobil ist;
- $J_0$ und $J_1$ die Verzögerung oder den Vorlauf des ersten Elements angeben, das auf der Grundlage des Indikators $j$ in die Berechnung einbezogen wird;
- $K_0$, $K_1$, $K_2$ die Verzögerung oder den Vorlauf des ersten Elements angeben, das in der entsprechenden lokalen Reihe zum Berechnen des angegebenen lokalen Durchschnitts verwendet wird;

wobei die Position des durch die Werte der Komponenten jedes der Vektoren $\delta_j$ angegebenen Punktes grafisch in zwei oder mehr Dimensionen dargestellt wird;

c) Berechnen des Abstands vom Massenschwerpunkt der Gruppe $\{\delta_J\}_{J=1,\ldots,M-N}$ zu einem Koordinatenursprung, der bestimmt ist durch:

$$\Phi_N = \left[ \sum_{k=1}^{N-1} \left( \sum_{i;N}^{M-N+1} \delta_{i,k} \right)^2 \right]^{1/2};$$

wobei $\delta_{i,k}$ die Komponente $k$ des Vektors $\delta_i$ ist und die Schreibweise der Summe $\sum_{i;N}^{M-N+1} \delta_{i,k}$ N mal N Sprünge in dem Indikator $i$; angibt;

d) visuelles Darstellen des Abstands vom Massenschwerpunkt in einem Koordinatensystem durch visuelle Darstellungsmittel.

2. System nach Anspruch 1, wobei die Mittel zur Berechnung und visuellen Darstellung geeignet sind, einen zusätzlichen Schritt des Vergleichens der gewonnenen nützlichen Daten mit Verhaltensmustern durchzuführen, die einer Vektorfunktion A = $\{a_j\}_{j=1,...,N}$ entsprechend der Parametrierung einer Herzsequenz zugeordnet sind, wobei die Elemente $a_j$ feste Werte oder Funktionen einer oder mehrerer Variablen sind und wobei ein zusätzlicher Schritt des Vergleichens der gewonnenen nützlichen Daten, die mit der Funktion A gewonnen wurden, gemäß den folgenden Schritten durchgeführt wird:

a) Berechnen des allgemeinen Winkels $\theta_i$, dessen Kosinus bestimmt ist durch:

$$\cos(\theta_i) = \frac{A \cdot \delta_i}{\|A\| \cdot \|\delta_i\|},$$

wobei das Symbol $\|\ \|$ die allgemeine Norm des Vektors in $N$ Dimensionen ist, sodass:

$$\|A\| = \left( \sum_{j=1}^{N} a_j^2 \right)^{1/2};$$

b) Berechnen der Anzahl von Ereignissen $M'$ sodass der Winkel $\theta_i$ kleiner als eine vorgegebene Toleranz $\varepsilon$ ist, wobei $0 < \varepsilon < 1$, sodass die Funktion $A$ in ihrem Existenzraum genutzt wird, um die Ereignisse zu finden, bei denen $\theta_i < \varepsilon$;
c) Berechnen des Koeffizienten $M'/M$;
d) visuelles Darstellen der berechneten Daten.

3. System nach dem vorhergehenden Anspruch, wobei die Berechnung der Reihe $\{\delta_j\}_{j=1,...,M-N}$ aufeinanderfolgender Vektoren mit $N$ Dimensionen oder Komponenten gemäß den folgenden Definitionen von Parametern durchgeführt wird:

$$\begin{cases} m = 0 \\ N = 5 \\ N_0 = 5 \\ N_1 = 5 \\ N_2 = 5 \\ \varepsilon_0 = 1 \\ \varepsilon_1 = 1 \\ \varepsilon_2 = 0 \\ \varsigma_0 = 1 \\ \varsigma_1 = 1 \\ J_0 = 0 \\ J_1 = 0 \\ K_0 = 0 \\ K_1 = 0 \\ K_2 = 0 \end{cases},$$

sodass die Definition der Variabilität in den M Intervallen lautet:

$$\delta_j = \left\{ X_{j+k} \langle X \rangle_{N,j}^{-1} - 1 \right\}_{k=0,\dots,N-1}.$$

**4.** System nach dem vorhergehenden Anspruch, wobei die Mittel zur Berechnung und Datendarstellung zum Durchführen der folgenden Schritte geeignet sind:

a) Berechnen Anzahl von Ereignissen anhand der Reihe $\{X_i\}_{i=1,\dots,M}$ mit der speziellen Definition des Vektors $A = t\{(N+1)/2 - J\}_{j=1,\dots,N}$, wobei $N > 1$;

b) Verwenden des Indikators $m_{s1}/M$, wobei $m_{s1} = M'$, der im vorhergehenden Punkt berechnet wurde, zum Bestimmen des Vorhandenseins von Verhaltensmustern, die der Vektorfunktion $A$ zugehörig sind;

c) visuelles Darstellen der berechneten Daten.

**5.** System nach Anspruch 3, wobei die Mittel zur Berechnung und Datendarstellung zum Durchführen der folgenden Schritte geeignet sind:

a) Berechnen der Anzahl von Ereignissen $M'$ anhand der Reihe $\{X_i\}_{i=1,\dots,M}$ mit der speziellen Definition des Vektors $A_N = t\{\sin(2\pi j)/N\}_{j=1,\dots,N}$, wobei N von $N = 3$ bis $N = 12$ reichen kann, was einer sinusförmigen Modulation des Herzrhythmus in Kombination mit dem Atemrhythmus entspricht, wobei t einen beliebigen Wert aufweisen kann;

b) Verwenden des Indikators $m_{s2}/M$, wobei $m_{S2} = M'$, der im vorangehenden Punkt berechnet wurde, zum Bestimmen des Vorhandenseins von Verhaltensmustern, die der Vektorfunktion A zugehörig sind;

c) visuelles Darstellen der berechneten Daten.

**6.** System nach Anspruch 3, wobei die Mittel zur Berechnung und Datendarstellung zum Durchführen der folgenden Schritte geeignet sind:

a) Berechnen des Koeffizienten $M'/M$ anhand der Reihe $\{X_i\}_{i=1,\dots,M}$ mit der Definition des Vektors

$$A_N = t\left\{-1, 1, \frac{0,\dots,0}{N}\right\}$$

, wobei $N$ von $N = 1$ bis $N = 20$ reichen kann, was einem kompensierten ektopischen Herzschlag entspricht, wobei $t$ einen beliebigen Wert aufweisen kann;

b) Verwenden des Indikators $m_E/M$, wobei $m_E = M'$, der im vorhergehenden Schritt berechnet wurde, zum Bestimmen des Vorhandenseins von Verhaltensmustern, die der Vektorfunktion $A_N$ zugehörig sind;

c) visuelles Darstellen der berechneten Daten.

**7.** System nach Anspruch 3, wobei die folgenden Schritte zusätzlich durchgeführt werden:

a) Berechnen des Koeffizienten $M'/M$ anhand der Reihe $\{X_i\}_{i=1,\dots,M}$ mit der speziellen Definition des Vektors

$$A_N = t\left\{N, \frac{-1,\dots,-1}{N}\right\}$$

, wobei TV von $N = 2$ bis $N = 20$, was einer regulären paroxysmalen Tachykardie entspricht und wobei t einen beliebigen Wert aufweisen kann;

b) Verwenden des Indikators $m_{TP}/M$, wobei $m_{TP} = M'$, der im vorhergehenden Punkt berechnet wurde, zum Bestimmen des Vorhandenseins von Verhaltensmustern, die der Vektorfunktion $A_N$ zugehörig sind;

c) visuelles Darstellen der berechneten Daten.

**8.** System nach einem der vorhergehenden Ansprüche, wobei die Komponente des Herz-"pQRSt"-Komplexes das RR-Intervall eines Elektrokardiogramms ist.

**9.** System nach einem der vorhergehenden Ansprüche, wobei das Aufzeichnen eine Anzahl $M$ von aufeinanderfolgenden Zeitintervallen $\{X_i\}_{i=1,\dots,M}$, die Zyklen einer Komponente eines "pQRSt"-Komplexes entsprechen, mit einer höheren Genauigkeit als 0,01 % des Mittelwerts der Zykluszeit durchgeführt wird.

**10.** System nach Anspruch 3, wobei $0 < \varepsilon < 0,1$.

**11.** System nach einem der Ansprüche 6 bis 8, wobei 11 oder -1 ist.

**Revendications**

1. Système d'obtention de données associées à un modèle de variabilité de la fréquence cardiaque (HRV), comprenant :

   a1) des moyens de mesure et d'enregistrement des données d'un électrocardiogramme, conçus pour mesurer et enregistrer un nombre $M$ d'intervalles de temps consécutifs $\{X_i\}_{i=1,\dots,M}$ correspondant aux cycles d'une ou plusieurs composantes d'un complexe cardiaque « pQRSt » d'un électrocardiogramme, avec une précision égale ou supérieure à 10 % de la valeur moyenne du temps de cycle, et $M$ étant supérieur à 2 ;
   a2) des moyens de calcul et de représentation visuelle, conçus pour effectuer des calculs sur les données enregistrées par les moyens de mesure et d'enregistrement de données, et représenter visuellement lesdites données ;

   **caractérisé en ce que** les moyens de calcul et de représentation des données sont conçus pour réaliser les étapes suivantes :

   b) calculer la variabilité sur lesdits $M$ intervalles d'une séquence de vecteurs consécutifs $\{\delta_J\}_{J=1,\dots,M-N}$ de $N$ composantes, selon l'algorithme ou la transformation définie par l'expression :

   $$\delta_j = \left\{ \sum_{n=0}^{m} \binom{m}{n} (-1)^n \left( \langle X \rangle_{N_0, j\cdot\varsigma_0 + K_0}^{-1} X_{j+n+k\cdot\varepsilon_0+J_0} - \varepsilon_1 \langle X \rangle_{N_1, j\cdot\varsigma_1+K_1}^{-1} \langle X \rangle_{N_2, j+n+k\cdot\varepsilon_2+K_2} \right) \right\}_{k=J_1,\dots,J_1+N-1},$$

   et la notation suivante :

   $$\langle X \rangle_{L,l} = L^{-1} \sum_{h=0}^{L-1} X_{l+h}, \text{ avec } \binom{m}{n} = \frac{m!}{n!(m-n)!},$$

   où les paramètres suivants sont des entiers et leur sélection détermine la forme finale de la transformation mentionnée :

   $$\{m, N, N_0, N_1, N_2, \varepsilon_0, \varepsilon_1, \varepsilon_2, \varsigma_0, \varsigma_1, J_0, J_1, K_0, K_1, K_2\},$$

   où en plus :

   - $m$ est un indicateur naturel représentant l'ordre de la variation discrète qui est calculée ;
   - $N$ est la dimension ou le nombre de composantes de chaque vecteur $\delta_J$, où $N \geq 2$ ;
   - $N_0, N_1$, et $N_2$ indiquent le nombre de valeurs qui sont utilisées pour calculer la moyenne locale indiquée correspondante dans la formule générale de l'algorithme ;
   - $\varepsilon_0, \varepsilon_1, \varepsilon_2$ ont des valeurs binaires 0 ou 1, et indiquent si les éléments correspondants sont, respectivement, fixes ou mobiles dans le calcul de chacune des composantes du vecteur $\delta_J$ ;
   - $\varsigma_0, \varsigma_1$ ont des valeurs binaires 0 ou 1, et indiquent si la moyenne locale est, respectivement, fixe ou mobile ;
   - $J_0$ et $J_1$ indiquent le retard ou l'avance du premier élément qui est pris en compte dans le calcul en fonction de l'indicateur $j$ ;
   - $K_0, K_1, K_2$ indiquent le retard ou l'avance du premier élément qui est pris dans la série locale correspondante pour le calcul de la moyenne locale indiquée ;

   dans lequel la position du point indiquée par les valeurs des composantes de chacun des vecteurs $\delta_j$ est représentée graphiquement en deux dimensions ou plus ;
   c) calculer la distance du centre de masse du groupe $\{\delta_j\}_{j=1,\dots,M-N}$ à une origine de coordonnées, telle que déterminée par :

$$\Phi_N = \left[ \sum_{k=1}^{N-1} \left( \sum_{i;N}^{M-N+1} \delta_{i,k} \right)^2 \right]^{1/2} ;$$

où $\delta_{i,k}$ est le composant $k$ du vecteur $\delta_i$, et la notation de la somme $\sum_{i;N}^{M-N+1} \delta_{i,k}$ indique N par N sauts dans l'indicateur $i$ ;

d) représenter visuellement ladite distance du centre de masse dans un système de coordonnées, par le biais des moyens de représentation visuelle.

2. Système selon la revendication 1, dans lequel les moyens de calcul et de représentation visuelle sont conçus pour réaliser une étape supplémentaire de comparaison des données utiles obtenues avec des modèles de comportement associés à une fonction vectorielle $A=\{a_j\}_{j=1,\ldots,N}$, correspondant au paramétrage d'une séquence cardiaque, où les éléments $a_j$ sont des valeurs fixes ou des fonctions d'une ou plusieurs variables, et où une étape supplémentaire de comparaison des données utiles obtenues avec ladite fonction A est réalisée selon les étapes suivantes :

a) calculer l'angle général $\theta_i$, dont le cosinus est déterminé par :

$$\cos(\theta_i) = \frac{A \cdot \delta_i}{\|A\| \cdot \|\delta_i\|},$$

où le symbole $\| \|$ est la norme générale d'un vecteur dans $N$ dimensions, tel que :

$$\|A\| = \left( \sum_{j=1}^{N} a_j^2 \right)^{1/2} ;$$

b) calculer le nombre d'événements $M'$ tel que l'angle $\theta_i$ est inférieur à une tolérance prédéterminée $\varepsilon$, où $0 < \varepsilon < 1$, tel que la fonction A est explorée dans son espace d'existence afin de trouver lesdits événements dans lesquels $\theta_i < \varepsilon$ ;

c) calculer le coefficient $M'/M$ ;

d) représenter visuellement les données calculées.

3. Système selon la revendication précédente, dans lequel le calcul de la série $\{\delta_j\}_{j=1,\ldots,M-N}$ de vecteurs consécutifs de $N$ dimensions ou composantes est réalisé selon les définitions de paramètres suivantes :

$$\left\{ \begin{array}{l} m = 0 \\ N = 5 \\ N_0 = 5 \\ N_1 = 5 \\ N_2 = 5 \\ \varepsilon_0 = 1 \\ \varepsilon_1 = 1 \\ \varepsilon_2 = 0 \\ \varsigma_0 = 1 \\ \varsigma_1 = 1 \\ J_0 = 0 \\ J_1 = 0 \\ K_0 = 0 \\ K_1 = 0 \\ K_2 = 0 \end{array} \right\},$$

tel que la définition de la variabilité sur lesdits M intervalles est :

$$\delta_j = \left\{ X_{j+k} \langle X \rangle_{N,j}^{-1} - 1 \right\}_{k=0,\ldots,N-1}.$$

4. Système selon la revendication précédente, dans lequel les moyens de calcul et de représentation des données sont conçus pour réaliser les étapes suivantes :

   a) calculer le nombre d'événements sur la base de la série $\{X_i\}_{i=1,\ldots,M}$ avec la définition précise du vecteur $A = t\{(N+1)/2 - j\}_{j=1,\ldots,N}$, où $N > 1$ ;
   b) utiliser l'indicateur $m_{s1}/M$, avec $m_{s1}=M'$ calculé au point précédent, pour déterminer l'existence de modèles de comportement associés à la fonction vectorielle $A$ ;
   c) représenter visuellement les données calculées.

5. Système selon la revendication 3, dans lequel les moyens de calcul et de représentation des données sont conçus pour réaliser les étapes suivantes :

   a) calculer le nombre d'événements $M'$ sur la base de la série $\{X_i\}_{i=1,\ldots,M}$ avec la définition précise du vecteur $A_N = t\{\sin(2\pi j)/N\}_{j=1,\ldots,N}$, où N peut aller de $N = 3$ à $N = 12$, correspondant à une modulation sinusoïdale du rythme cardiaque combinée au rythme respiratoire, où $t$ peut avoir n'importe quelle valeur ;
   b) utiliser l'indicateur $m_{s2}/M$, avec $m_{s2} = M'$ calculé au point précédent, pour déterminer l'existence de modèles de comportement associés à la fonction vectorielle $A$ ;
   c) représenter visuellement les données calculées.

6. Système selon la revendication 3, dans lequel les moyens de calcul et de représentation des données sont conçus pour réaliser les étapes suivantes :

   a) calculer le coefficient $M'/M$ sur la base de la série $\{X_i\}_{i=1,\ldots,M}$ avec la définition du vecteur

   $$A_N = t\left\{ -1, 1, \frac{0,\ldots,0}{N} \right\},$$ où $N$ peut aller de $N = 1$ à $N = 20$, correspondant à un battement ectopique compensé, et où t peut avoir n'importe quelle valeur ;
   b) utiliser l'indicateur $m_E/M$, avec $m_E=M'$ calculé à l'étape précédente, pour déterminer l'existence de modèles de comportement associés à la fonction vectorielle $A_N$ ;
c) représenter visuellement les données calculées.

7. Système selon la revendication 3, dans lequel les étapes suivantes sont en outre exécutées :

a) calculer le coefficient $M'/M$ sur la base de la série $\{X_i\}_{i=1,\ldots,M}$ avec la définition précise du vecteur

$$A_N = t\left\{N, \frac{-1,\ldots,-1}{N}\right\}$$ , où TV peut aller de $N = 2$ à $N = 20$, correspondant à une tachycardie paroxystique régulière, et où $t$ peut avoir n'importe quelle valeur ;
b) utiliser l'indicateur $m_{TP}/M$, avec $m_{TP}=M'$ calculé au point précédent, pour déterminer l'existence de modèles de comportement associés à la fonction vectorielle $A_N$ ;
c) représenter visuellement les données calculées.

8. Système selon l'une quelconque des revendications précédentes, dans lequel la composante du complexe cardiaque « pQRSt » est l'intervalle RR d'un électrocardiogramme.

9. Système selon l'une quelconque des revendications précédentes, dans lequel l'enregistrement d'un nombre $M$ d'intervalles de temps consécutifs $\{X_i\}_{i=1,\ldots,M}$, correspondant aux cycles d'une composante d'un complexe cardiaque « pQRSt » est réalisé avec une précision supérieure à 0,01 % de la valeur moyenne du temps de cycle.

10. Système selon la revendication 3, dans lequel $0 < \varepsilon < 0,1$.

11. Système selon l'une quelconque des revendications 6 à 8, dans lequel t vaut 1 ou -1.

FIG. 1

FIG. 2

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4934374 A, Ostlund
- US 493374 A
- US 20050171447 A1, Esperer
- GB 2015050429 W
- WO 2015033244 A1
- WO 2015052609 A1
- US 20050171447 A

**Non-patent literature cited in the description**

- **GAÑÁN-CALVO et al.** Universal structures of normal and pathological heart rate variability. *Sci. Rept.,* 25 February 2016, vol. 6 (1), 1-11